# EUROPÄISCHE PATENTANMELDUNG

(11) **EP 4 772 151 A1**
(43) Veröffentlichungstag der Anmeldung: **08.07.2026**
(21) Anmeldenummer: 26150431.0
(22) Anmeldetag: 06.01.2026
(51) Int. Cl.: A61F 13/472

(54) **HYGIENEPRODUKT MIT BLICKSCHUTZELEMENT**

(30) Priorität: 07.01.2025 DE 102025100327; 07.01.2025 DE 202025100408 U
(71) Anmelder: Caysuyu, Nazan, 76764 Rheinzabern (DE)
(72) Erfinder: Caysuyu, Nazan, 76764 Rheinzabern (DE)
(74) Vertreter: Boukis, Andreas

(57) **Zusammenfassung**

Vorgeschlagen wird ein Hygieneprodukt (1), insbesondere eine Monatsbinde oder eine Slipeinlage, zum Tragen an einem Bekleidungsstück, welches den Genitalbereich einer Person bedeckt. Das Hygieneprodukt (1) umfasst einen Grundkörper (2), welcher eine erste elastische Verformbarkeit aufweist, und ein Blickschutzelement (3), welches eine zweite elastische Verformbarkeit aufweist, die geringer ist als die erste elastische Verformbarkeit. Das Hygieneprodukt (1) ist dazu ausgebildet, bei dem Tragen an dem Bekleidungsstück, ein bei Draufsicht auf das Bekleidungsstück glattes Aussehen über dem Genitalbereich bereitzustellen.

## Beschreibung

Die Erfindung betrifft ein Hygieneprodukt gemäß Anspruch 1 zum Tragen an einem Bekleidungsstück, welches den Genitalbereich einer Person bedeckt. Des Weiteren betrifft die Erfindung ein Blickschutzelement gemäß Anspruch 8, ein Set gemäß Anspruch 10, ein Verfahren zum Herstellen eines Hygieneprodukts gemäß Anspruch 11, die Verwendung eines Hygieneprodukts gemäß Anspruch 12 und die Verwendung eines Blickschutzelements gemäß Anspruch 13.

Hygieneprodukte wie Monatsbinden oder Slipeinlagen sind beispielsweise aus
EP 0 156 257 A2 vorbekannt. Hygieneprodukte wie Monatsbinden haben viele Vorteile und bieten zum Beispiel zuverlässigen Schutz während der Menstruation, indem sie Menstruationsblut aufnehmen. Slipeinlagen schützen die Unterwäsche bei leichteren Flüssigkeitsabsonderungen oder als Ergänzung zu Tampons oder Menstruationstassen. Viele vorbekannte Hygieneprodukte sind bereits so konzipiert, dass sie angenehm zu tragen sind, atmungsaktiv sind und sich eng an die Körperform anpassen. Die enge Anpassung an die Körperform ist häufig ein gewünschter Effekt, damit sich die äußeren Konturen des Hygieneproduktes nicht an der darüber liegenden Bekleidung abzeichnen und somit nicht erkannt wird, dass die Person das Hygieneprodukt trägt. Das enge Anpassung an die Körperform kann durch ein Aufweichen des Hygieneprodukts beim Tragen, beispielsweise durch Feuchtigkeit und die Körpertemperatur, noch weiter verstärkt werden. Das enge Anpassen an die Körperform führt neben den gewünschten Effekten aber auch zu unerwünschten Nebeneffekten, wie nachfolgend noch näher erläutert wird.

Ausgehend von den bereits vorbekannten Hygieneprodukten liegt der vorliegenden Erfindung die Aufgabe zu Grunde, ein verbessertes Hygieneprodukt anzugeben. Das verbesserte Hygieneprodukt adressiert neben bereits bekannten technischen Problemen von Hygieneprodukten wie einer verbesserten Saugfähigkeit auch einen völlig anderen Problemkreis, nämlich das Bereitstellen eines visuell neutralen und unauffälligen Aussehens von Bekleidungsstücken, an denen das Hygieneprodukt getragen wird. Diese Aufgabe wird durch die vorliegende Erfindung gelöst. Die Erfindung ist durch die Gegenstände mit den Merkmalen der darauf gerichteten unabhängigen Ansprüche definiert. Vorteilhafte Ausführungsformen und Weiterbildungen der Erfindung sind Gegenstand von Unteransprüchen und der nachfolgenden Beschreibung.

Das erfindungsgemäße Blickschutzelement und bevorzugte Weiterbildungen desselben sind vorzugsweise für eine Anwendung in dem erfindungsgemäßen Hygieneprodukt, dem erfindungsgemäßen Verfahren und der erfindungsgemäßen Verwendung, sowie in bevorzugten Weiterbildungen derselben, ausgebildet und vorgesehen. Ebenso sind auch das erfindungsgemäße Hygieneprodukt und das erfindungsgemäße Blickschutzelement, sowie bevorzugte Weiterbildungen derselben für den Einsatz in dem erfindungsgemäßen Set, dem erfindungsgemäßen Verfahren und der erfindungsgemäßen Verwendung, sowie in bevorzugten Weiterbildungen derselben, ausgebildet und vorgesehen. Alle Merkmale der beschriebenen Gegenstände und auch der beanspruchten Gegenstände sind sowohl isoliert als auch in Kombination miteinander verwendbar, untereinander kompatibel und zur Weiterbildung untereinander vorgesehen und einsetzbar, sofern sich kein logischer Widerspruch ergibt, und hiermit dahingehend offenbart. Die bloße Tatsache, dass bestimmte Merkmale in unterschiedlichen Ansprüchen genannt werden, bedeutet nicht, dass eine Kombination dieser Merkmale nicht vorteilhaft sein kann.

Im Folgenden ist jede Bezugnahme auf einen Gegenstand oder ein Merkmal (einschließlich der unbestimmten Artikel "ein" und "eine" und der bestimmten Artikel "der", "die", "das"), zwei Gegenstände oder zwei Merkmale, oder eine andere Anzahl von Gegenständen oder Merkmalen, sofern nicht ausdrücklich etwas anderes erwähnt wird oder sich ein logischer Widerspruch ergibt, so zu verstehen, dass das Vorhandensein weiterer solcher Gegenstände und Merkmale von der Erfindung nicht ausgeschlossen wird, sondern von der Erfindung auch umfasst ist. Die Worte "umfassend", "aufweisend", "enthaltend" und "mit" schließen weitere Gegenstände, Merkmale, Elemente oder Schritte nicht aus. Die Verwendung von "oder" bedeutet "und/oder", sofern nicht anders angegeben. Die Verwendung von "und/oder" bedeutet, dass die vorangehenden und nachfolgenden Begriffe zusammen oder getrennt betrachtet werden können. Zur Veranschaulichung, aber nicht als Einschränkung, kann "X und/oder Y" "X" oder "Y" oder "X und Y" bedeuten. Wenn ein Wertebereich angegeben ist, umfasst dieser Bereich den Anfangswert und den Endwert sowie jeden Wert oder Wertebereich dazwischen, sofern nicht ausdrücklich etwas anderes angegeben ist. Zum Beispiel bedeutet "von 0,2 bis 0,5" 0,2, 0,3, 0,4, 0,5; dazwischen liegende Bereiche wie 0,2-0,3, 0,3-0,4, 0,2-0,4; dazwischen liegende Inkremente wie 0,25, 0,35, 0,225, 0,335, 0,49; dazwischen liegende Inkrementbereiche wie 0,26-0,39; und dergleichen. Die Bezugszeichen in den Ansprüchen sind nicht als einschränkend zu verstehen, sondern dienen lediglich der besseren Lesbarkeit der Ansprüche.

Gemäß einem ersten Aspekt betrifft die Erfindung ein Hygieneprodukt, insbesondere eine Monatsbinde oder eine Slipeinlage. Dieses Hygieneprodukt ist zum Tragen an einem Bekleidungsstück, welches den Genitalbereich einer Person bedeckt, insbesondere an Unterwäsche vorgesehen. Das Hygieneprodukt weist einen Grundkörper auf. Der Grundkörper weist eine erste elastische Verformbarkeit auf. Zudem weist das Hygieneprodukt ein Blickschutzelement auf. Dieses Blickschutzelement gemäß des ersten Aspekts der Erfindung weist eine zweite elastische Verformbarkeit auf. Die zweite elastische Verformbarkeit ist geringer als die erste elastische Verformbarkeit. Das Hygieneprodukt ist dazu ausgebildet, bei dem Tragen an dem Bekleidungsstück, ein bei Draufsicht auf das Bekleidungsstück glattes Aussehen über dem Genitalbereich bereitzustellen.

Gemäß einem zweiten Aspekt betrifft die Erfindung ein Blickschutzelement für ein Hygieneprodukt gemäß dem ersten Aspekt der Erfindung und/oder für ein Bekleidungsstück. Bei dem Blickschutzelement gemäß des zweiten Aspekts der Erfindung ist die zweite elastische Verformbarkeit des Blickschutzelements geringer als die erste elastische Verformbarkeit des Hygieneprodukts und/oder geringer als eine elastische Verformbarkeit des Bekleidungsstücks.

Gemäß einem dritten Aspekt betrifft die Erfindung ein Set umfassend ein Hygieneprodukt, insbesondere eine Monatsbinde oder eine Slipeinlage, und ein Blickschutzelement gemäß dem zweiten Aspekt. Das Set umfasst ferner ein Befestigungselement, insbesondere einen Klebestreifen, zur Befestigung des Blickschutzelements an dem Hygieneprodukt und/oder an einem Bekleidungsstück; und/oder das Blickschutzelement weist an einer dem Hygieneprodukt und/oder dem Bekleidungsstück zuzuwendenden Oberfläche ein Haftmittel auf und/oder das Blickschutzelement besteht aus einem haftenden Material.

Gemäß einem vierten Aspekt betrifft die Erfindung ein Verfahren zum Herstellen eines Hygieneprodukts mit einem Blickschutzelement, insbesondere einer Monatsbinde oder einer Slipeinlage, mit den Schritten:
- Bereitstellen eines Grundkörpers
- Einbringen oder Aufbringen eines fluiden Materials zwischen zwei Schichten des Grundkörpers oder applizieren des fluiden Materials auf einer kleidungsseitigen Oberfläche des Grundkörpers oder auf einer körperseitigen Oberfläche des Grundkörpers
- Ausbilden eines Blickschutzelements durch Verfestigen des fluiden Materials.

Gemäß einem fünften Aspekt betrifft die Erfindung die Verwendung eines Hygieneprodukts, insbesondere einer Monatsbinde oder einer Slipeinlage, an einem Bekleidungsstück, welches den Genitalbereich einer Person bedeckt, zum Bereitstellen eines bei Draufsicht auf das Bekleidungsstück glatten Aussehens des Genitalbereichs.

Gemäß einem sechsten Aspekt betrifft die Erfindung die Verwendung eines Blickschutzelements an einem Hygieneprodukt, insbesondere einer Monatsbinde oder einer Slipeinlage, und/oder an einem Bekleidungsstück, welches den Genitalbereich einer Person bedeckt, zum Bereitstellen eines bei Draufsicht auf das Bekleidungsstück glatten Aussehens des Genitalbereichs.

Die Erfindung betrifft insbesondere den Problemkreis des Abzeichnens von Konturen des Genitalbereichs von Personen, die enganliegende Bekleidungsstücke über dem Genitalbereich tragen. Dabei kann sich das Bekleidungsstück in zumeist unerwünschter Weise derart an den Schritt von Personen mit einer Vulva anpassen, dass die Form der Vulva, insbesondere die Kontur der Schamlippen, durch das Bekleidungsstück hindurch von außen betrachtet sichtbar ist. Diese unerwünschte Entblößung wird manchmal euphemistisch als "Cameltoe" bezeichnet und beschreibt eine unerwünschte "W"-Form. Eine solche unerwünschte Entblößung kann durch eine Naht des Bekleidungsstücks direkt in der Mitte des Schritts noch weiter verstärkt werden. Ebenso ist es von Personen mit einer anderen Genitalmorphologie, beispielsweise einem Penis und/oder einem Hodensack, zumeist unerwünscht, dass sich die Konturen des Genitalbereichs an darüberliegenden Bekleidungsstücken abzeichnen. Vorteilhafterweise werden diese Probleme durch die vorliegende Erfindung gelöst. Besonders vorteilhafterweise wird durch die vorliegende Erfindung außerdem ein besonders sicherer Sitz eines am Körper getragenen Hygieneprodukts bereitgestellt. Außerdem sorgt das Blickschutzelement für eine zusätzliche thermische Isolation und schützt somit vor Wärmeverlust. Bevorzugt können das Hygieneprodukt und/oder das Blickschutzelement Wärme im Genitalbereich speichern, was besonders in kalten Klimazonen von Vorteil ist. Darüber hinaus können das Hygieneprodukt und/oder das Blickschutzelement als Barriere gegen Feuchtigkeit dienen, sodass Flüssigkeiten nicht eindringen und/oder austreten können, wodurch sich der Tragekomfort vorteilhaft erhöht. Das Blickschutzelement kann optional zusätzliche Feuchtigkeit aufnehmen und damit die Saugfähigkeit des Hygieneprodukts verbessern.

Bei einer bevorzugten Ausführungsform kann das "Hygieneprodukt" neben den bereits genannten Monatsbinden und Slipeinlagen insbesondere auch als Windel und/oder als Inkontinenzeinlage ausgebildet sein. Bevorzugt ist das Hygieneprodukt ein Einwegprodukt und reduziert vorteilhafterweise das Risiko von Infektionen, da es nach der Nutzung entsorgt wird. Bei einer bevorzugten Ausführungsform ist das "Bekleidungsstück" insbesondere ein Unterbekleidungsstück für die untere Körperhälfte. Der Begriff Bekleidungsstück umfasst neben Unterwäsche auch Sportbekleidung und/oder Leggins und/oder Yoga-Hosen und/oder Stumpfhosen und/oder Bikinis und/oder Badeanzüge und/oder Badehosen und/oder Radlerhosen und/oder Reithosen und/oder Shorts und/oder Jogginghosen und/oder Laufhosen und/oder Bodysuits.

Bei einer bevorzugten Ausführungsform weist der "Grundkörper" bevorzugt die körperseitige Oberfläche und die gegenüberliegende kleidungsseitige Oberfläche auf. Bevorzugt ist die körperseitige Oberfläche dem Genitalbereich zugewandt. Die kleidungsseitige Oberfläche ist bevorzugt dem Bekleidungsstück zugewandt. Der Grundkörper umfasst vorzugsweise ein Cellulosematerial oder besteht aus dem Cellulosematerial. Alternativ oder ergänzend umfasst der Grundkörper vorzugsweise ein Vliesmaterial oder besteht aus dem Vliesmaterial. Der Grundkörper umfasst bevorzugt ein synthetisches Material oder besteht aus dem synthetischen Material. Das synthetische Material ist bevorzugt ausgewählt aus der Gruppe: Polyethylen, und/oder Polypropylen, und/oder Polyamid, und/oder Polyester, und/oder Polyacrylat, und/oder Polyethylenterephthalat, und/oder Polylactid, und/oder Viskose, und/oder Lyocell, und/oder Seide. Das synthetische Material ist vorzugsweise faserförmig.

Der Grundkörper weist vorzugsweise ein Absorptionselement auf, welches besonders vorzugsweise dazu ausgebildet ist, Flüssigkeiten zu aufzunehmen und/oder zu binden. Das Absorptionselement besteht bevorzugt aus einem Absorptionsmaterial, welches Flüssigkeiten wie insbesondere Körperflüssigkeiten chemisch und/oder physikalisch bindet. Das Absorptionsmaterial umfasst vorzugsweise das Cellulosematerial, insbesondere ein saugfähiges Cellulosematerial, oder besteht daraus. Das Absorptionsmaterial umfasst vorzugsweise ein Polyacrylat oder besteht aus dem Polyacrylat. Das Absorptionsmaterial kann ein teilchenförmiges Material, beispielsweise Gelperlen, mit einer durchschnittlichen Teilchengröße von 10 µm bis 1000 µm sein. Bevorzugt ist das Absorptionselement mit dem synthetischen Material überzogen oder davon umgeben, insbesondere um das Cellulosematerial mit den saugfähigen Gelperlen zu verbinden. Vorteilhafterweise können Flüssigkeiten dadurch besonders gut aufgenommen und gebunden werden.

Der Grundkörper umfasst bevorzugt eine Vielzahl von Schichten, besonders bevorzugt zumindest eine erste Schicht und eine zweite Schicht. Das Blickschutzelement kann bevorzugt zwischen zwei beliebigen oder an einer äußeren Oberfläche des Grundkörpers angeordnet sein. Die erste Schicht kann die körperseitige Oberfläche umfassen. Die zweite Schicht kann die kleidungsseitige Oberfläche umfassen. Der Grundkörper kann eine dritte Schicht aufweisen, welche bevorzugt zwischen der ersten Schicht und der zweiten Schicht angeordnet ist und besonders bevorzugt angrenzend an der zweiten Schicht angeordnet ist. Der Grundkörper kann eine vierte Schicht umfassen, welche bevorzugt zwischen der ersten Schicht und der zweiten Schicht angeordnet ist, besonders bevorzugt zwischen der dritten Schicht und der zweiten Schicht und höchst bevorzugt angrenzend an der dritten Schicht angeordnet ist. Dabei können die erste Schicht und/oder die dritte Schicht das synthetische Material, insbesondere faserförmig, enthalten oder daraus bestehen. Vorteilhafterweise sind die erste Schicht und/oder die dritte Schicht dazu ausgebildet Flüssigkeit schnell abzuleiten, insbesondere zu dem Absorptionselement, sodass die Haut angenehm trocken gehalten wird. Außerdem können die erste Schicht und/oder die dritte Schicht Textilmaterialien und/oder Baumwolle enthalten oder daraus bestehen. Vorteilhafterweise sind die erste Schicht und/oder die dritte Schicht ähnlich wie Wäsche und Unterwäsche verarbeitet. Bevorzugt ist zumindest eine Schicht, vorzugsweise ist die erste Schicht und/oder die zweite Schicht, oder es sind alle Schichten, aus einem gewebten Material gebildet.

Die vierte Schicht besteht aus dem Absorptionselement oder umfasst das Absorptionselement, wobei das Absorptionsmaterial insbesondere homogen oder inhomogen innerhalb der vierten Schicht dispergiert ist. Die vierte Schicht weist bevorzugt eine Oberfläche auf, welche der zweiten Schicht zugewandt ist, wobei an dieser Oberfläche geruchsneutralisierende Stoffe und/oder Parfümduftstoffe vorhanden sind. Vorteilhafterweise werden dadurch unangenehme Gerüche entfernt oder überdeckt. Die erste Schicht und/oder die zweite Schicht und/oder die dritte Schicht sind vorzugsweise frei von Absorptionsmaterial. Die zweite Schicht ist bevorzugt feuchtigkeitsdicht und gewährleistet vorteilhafterweise, dass keine Flüssigkeit nach außen dringt. Bevorzugt ist die zweite Schicht aus dem synthetischen Material gebildet, insbesondere in From einer geschlossenen Materialschicht. Die zweite Schicht kann frei von Cellulosematerial sein. Bevorzugt ist an der kleidungsseitigen Oberfläche der zweiten Schicht ein Klebstoff und/oder das Blickschutzelement vorgesehen und angeordnet. Vorteilhafterweise hat die kleidungsseitige Oberfläche einen Aufdruck, der darauf hinweist, dass die zweite Schicht bei korrekter Anwendung des Hygieneprodukts nicht mit der Haut in Kontakt kommt. Alternativ bevorzugt oder zusätzlich bevorzugt kann das Blickschutzelement dadurch bereitgestellt und/oder ausgebildet sein, dass zumindest eine der Schichten des Grundkörpers in einem bei bestimmungsgemäßer Anwendung der Körpervorderseite zuzuwendenden vorderen Bereich, insbesondere in Längsrichtung des Grundkörpers betrachtet, mit einer erhöhten Materialstärke und/oder steifer ausgebildet ist. Vorzugsweise kann die erste Schicht in dem vorderen Bereich dicker ausgeführt sein als in einem dem vorderen Bereich gegenüberliegenden hinteren Bereich, welcher insbesondere der Körperrückseite bestimmungsgemäß zuzuwenden ist. Bevorzugt kann die vierte Schicht und insbesondere das Absorptionselement, in dem vorderen Bereich verdickt sein, um das Blickschutzelement auszubilden und/oder derart ausgebildet sein, dass dort das Blickschutzelement angeordnet ist. Bevorzugt ist das jeweilige Material der übrigen Bereiche des Grundkörpers, insbesondere in dem hinteren Bereich, vergleichsweise dünner und/oder weicher. Hierdurch wird beim Tragen eine glatte, formstabile Oberfläche in dem vorderen Bereich erzielt, wodurch der Tragekomfort verbessert und ein unerwünschtes Verrutschen des Hygieneprodukts zuverlässig reduziert wird.

Der Grundkörper umfasst bevorzugt von 10% bis weniger als 70% Cellulosematerial und/oder von 10% bis weniger als 70% Absorptionsmaterial, wobei die Prozentsätze insbesondere Gewichtsprozentsätze auf der Basis des Trockengewichts des Grundkörpers sind. Insbesondere weist der Grundkörper eine Trockendichte und eine Nassdichte auf, wobei die Nassdichte von 5 Mal bis 20 Mal so groß ist wie die Trockendichte. Insbesondere beträgt die Trockendichte von 0,04 g/cm³ bis 0,3 g/cm³. Vorteilhafterweise ist der Grundkörper flexibel und ermöglicht eine Formanpassung. Vorzugsweise weist der Grundkörper Belüftungslöcher in einem Randbereich auf, sodass der Tragekomfort erhöht wird.

Bei einer bevorzugten Ausführungsform umfasst die "elastische Verformbarkeit" bevorzugt, dass die entsprechenden Gegenstände oder Elemente, wie insbesondere der Grundkörper, das Blickschutzelement und das Bekleidungsstück, in einem unbelasteten Zustand eine Grundform aufweisen und dazu ausgebildet sind, bei einer elastischen Verformung, beispielsweise unter Kraft- oder Druckeinwirkung, verformt zu werden und nach der elastischen Verformung, insbesondere nach Wegfallen des Drucks oder der Kraft die Grundform wieder einzunehmen. Eine geringere elastische Verformbarkeit meint vorzugsweise, dass bei gleicher Kraft- oder Druckeinwirkung eine geringere Verformung stattfindet. Beispielsweise wird bei einer Kraft- oder Druckeinwirkung auf das Hygieneprodukt der Grundkörper und/oder das Bekleidungsstück stärker verformt als das Blickschutzelement. In anderen Worten ist das Blickschutzelement bevorzugt steifer als der Grundkörper und/oder als das Bekleidungsstück. Die elastische Verformbarkeit kann anhand des Elastizitätsmoduls und/oder einer Spannungs-Dehnungs-Analyse und/oder einer Dynamisch-Mechanischen-Analyse und/oder einer anderen Methode, wie beispielsweise Ultraschallmessungen, bestimmt werden. Beispielsweise können dazu Zugversuche nach DIN EN ISO 527 / ASTM D638 und/oder Biegeversuche nach DIN EN ISO 178 / ASTM D790 und/oder Dynamisch-mechanische Analysen nach DIN EN ISO 6721-1 und/oder andere Verfahren zum Einsatz kommen.

Bei einer bevorzugten Ausführungsform umfasst das "glatte Aussehen" insbesondere ein Erscheinungsbild, bei dem die Konturen des Genitalbereichs nicht sichtbar sind, oder die Sichtbarkeit der Konturen des Genitalbereichs beim Tragen des Hygieneprodukts an dem Bekleidungsstück im Vergleich zum Tragen des Bekleidungsstücks ohne das Hygieneprodukts zumindest verringert ist. Das glatte Aussehen beschreibt also vorzugsweise eine glatte und unauffällige Passform der Belkleidung im Genitalbereich, insbesondere ohne sichtbare Konturen des Genitalbereichs an dem Bekleidungsstück. Das glatte Aussehen kann bevorzugt durch eine betrachtende Person oder durch Bildanalysen bei unterschiedlichen Lichtverhältnissen, insbesondere mit unterschiedlichen Lichtquellen, Lichtwinkeln und/oder Farbkontrasten ermittelt werden. Alternativ oder ergänzend bevorzugt kann das glatte Aussehen durch 3D-Scanns festgestellt werden. Vorteilhafterweise eignen sich das Hygieneprodukt und das Blickschutzelement für Alltagssituationen, wobei ein ästhetisches Erscheinungsbild bei figurbetonter Bekleidung wie engen Hosen, Röcken oder Leggings bewahrt wird. Auch bei der Anwendung in Kombination mit eleganter Abendgarderobe ist das glatte Aussehen besonders vorteilhaft. Außerdem ist im Speziellen auch die Anwendung für den Sport bevorzugt, wobei das Abzeichnen von unerwünschten Konturen bei körperlicher Aktivität wie Yoga, Fitnesstraining oder beim Joggen verhindert wird. Ebenso vorteilhaft ist der Einsatz im Beruf, wobei ein hoher Komfort und Diskretion auch bei längeren Tragezeiten wie zum Beispiel im Büro bereitgestellt wird. Ferner kommen auch Anwendungen im Bereich des Arbeitsschutzes in Betracht, da durch das Hygieneprodukt und das Blickschutzelement eine weitere mechanische Barriere zum Schutz des Genitalbereichs gegen unerwünschte Kraft- oder Stoffeinwirkungen bereitgestellt wird.

Bei einer bevorzugten Ausführungsform ist das "fluide Material" vorzugsweise ein fließfähiges Material, welches nach dem Verfestigen das Blickschutzelement ausbildet. Das fluide Material ist bevorzugt ausgewählt aus der Gruppe: Silikon, und/oder Polyurethan, und/oder Epoxidharz, und/oder UV-härtende Polymere, und/oder Wachs, insbesondere Mischwachs und/oder Paraffin, und/oder Hydrogel, und/oder Latex und/oder weitere Materialien des Blickschutzelements oder einer beliebigen Kombination der Materialien. Vorteilhafterweise ermöglicht die Auswahl eines fließfähigen Materials wie Silikon, Polyurethan, Epoxidharz oder UV-härtenden Polymeren eine einfache Verarbeitung und Anpassung an unterschiedliche Geometrien, wobei nach dem Verfestigen ein funktionales Blickschutzelement entsteht. Zudem erlaubt die breite Materialvielfalt eine gezielte Einstellung von Elastizität und mechanischer Beständigkeit.

Bei einer bevorzugten Ausführungsform erfolgt das "Einbringen" des fluiden Materials bevorzugt zwischen zwei Schichten des Grundkörpers. Dabei kann das fluide Material zwischen den beiden Schichten eingespritzt werden. Bei einer bevorzugten Ausführungsform erfolgt das "Aufbringen" des fluiden Materials bevorzugt durch ein Applizieren des fluiden Materials, beispielsweise durch ein Aufgießen oder ein Aufsprühen. Besonders bevorzugt wird das fluide Material auf der kleidungsseitigen Oberfläche des Grundkörpers appliziert. Alternativ kann das fluide Material auch auf eine andere Oberfläche, insbesondere die körperseitige Oberfläche aufgebracht werden. Vorzugsweise wird bei dem Aufgießen das fluide Material in eine passende Form gegossen. Optional wird der Grundkörper vor dem Aufgießen in die Form eingelegt. Durch das Aufsprühen werden vorteilhafterweise besonders dünne und gleichmäßige Schichten an fluidem Material appliziert. Zum Einbringen oder Aufbringen von fluidem Material kann beispielsweise ein Spritzgussverfahren und/oder ein 3D-Druckverfahren eingesetzt werden. Bei einer bevorzugten Ausführungsform erfolgt das "Verfestigen" des fluiden Materials vorzugsweise durch ein Aushärten und/oder ein Trocknen und/oder ein Quervernetzen des Materials. Bevorzugt wird das Verfestigen des Materials durch eine Bestrahlung durchgeführt, insbesondere durch eine Bestrahlung mit UV-Licht und/oder IR-Strahlung. Vorteilhafterweise erlaubt das Einbringen oder Aufbringen des fluiden Materials zwischen oder auf den Schichten des Grundkörpers eine flexible Fertigung mit anpassbarer Dicke und Struktur. Dadurch können sowohl sehr gleichmäßige als auch gezielt strukturierte Blickschutzelemente erzeugt werden, die sich über Verfahren wie Spritzguss oder 3D-Druck kosteneffizient herstellen lassen. Zudem ermöglicht die Verfestigung durch Aushärten, Trocknen oder Bestrahlung eine schnelle und prozesssichere Herstellung mit reproduzierbaren Materialeigenschaften.

Bei einer bevorzugten Ausführungsform kann das "Blickschutzelement" auch unabhängig von dem Hygieneprodukt eingesetzt werden, beispielsweise an dem Bekleidungsstück, bevorzugt an einer dem Genitalbereich zugewandten inneren Oberfläche des Bekleidungsstücks. Das Blickschutzelement kann bevorzugt direkt auf die dem Genitalbereich zugewandte Oberfläche des Bekleidungsstücks aufgebracht werden, besonders bevorzugt als fluides Material in gleicher Weise wie bei dem Applizieren auf den Grundkörper, wobei insbesondere nach dem Verfestigen das Blickschutzelement direkt an dem Bekleidungsstück angeordnet ist. Alternativ kann das Blickschutzelement, insbesondere in bereits fester Form, auf die dem Genitalbereich zugewandte Oberfläche des Bekleidungsstücks gelegt oder angeklebt werden. Das Einlegen oder Ankleben kann sowohl manuell als auch maschinell erfolgen. Sofern das an dem Bekleidungsstück aufgebrachte Blickschutzelement bevorzugt ein wasserlösliches Material aufweist oder daraus besteht, kann das Blickschutzelement vorteilhafterweise einfach von dem Bekleidungsstück abgelöst werden, beispielsweise durch ein Waschen des Bekleidungsstücks, insbesondere in einer handelsüblichen Waschmaschine. Bevorzugt ist ein einzelnes Blickschutzelement an dem Hygieneprodukt und/oder an dem Bekleidungsstück vorgesehen und/oder daran angeordnet. Alternativ bevorzugt können auch mehrere identische oder unterschiedliche Blickschutzelemente an dem Hygieneprodukt und/oder an dem Bekleidungsstück vorgesehen sein und/oder daran angeordnet sein.

Bei einer bevorzugten Weiterbildung umfasst das Blickschutzelement ein wasserlösliches Material oder besteht aus einem wasserlöslichen Material. Das "wasserlösliche Material" ist bevorzugt ausgewählt aus der Gruppe: Polyvinylalkohol, und/oder Polysaccharide, insbesondere modifizierte Polysaccharide, und/oder Polyethylenglykol, insbesondere Polyethylenglykol basierte Nanopartikel, oder einer beliebigen Kombination der Materialien. Das wasserlösliche Material ist vorzugsweise zudem ein thermo-responsives wasserlösliches Material, welches sich bei erhöhter Temperatur besser und/oder schneller in Wasser auflöst. Polyvinylalkohol hat die Vorteile, dass es sich besonders schnell in Wasser auflöst, medizinisch erprobt ist und biologisch abgebaut werden kann. Polysaccharide und modifizierte Polysaccharide haben neben einer guten Wasserlöslichkeit die Vorteile, dass sie in nachhaltiger Weise produziert werden können und biologisch abbaubar sind. Polyethylenglykol und insbesondere Polyethylenglykol basierte Nanopartikel eignen sich, um vorteilhafterweise Wirkstoffe oder Duftstoffe freizusetzen.

Bei einer bevorzugten Weiterbildung weist das Blickschutzelement einen Kernbereich und einen Randbereich auf. Bevorzugt umschließt der Randbereich den Kernbereich zumindest teilweise oder vollständig. Der Kernbereich weist vorzugsweise eine erste Materialstärke auf. Der Randbereich weist vorzugsweise eine zweite Materialstärke auf. Die erste Materialstärke ist vorzugsweise größer als die zweite Materialstärke. Bevorzugt ist die erste Materialstärke mehr als 10% größer als die zweite Materialstärke, besonderes bevorzugt mehr als 20%, höchst bevorzugt mehr als 30%. Vorzugsweise beträgt die erste Materialstärke von 0,1 mm bis 20 mm, besonders vorzugsweise von 0,5 mm bis 10 mm, höchst vorzugsweise von 1 mm bis 5 mm, weiter vorzugsweise von 1 mm bis 3 mm. Vorteilhafterweise ist das Blickschutzelement dadurch in dem Randbereich verformbarer als in dem Kernbereich. Dadurch lässt sich ein besonders hoher Tragekomfort erreichen. Gleichzeitig wird vorteilhafterweise durch den stabileren Kernbereich eine zuverlässige Blickschutzwirkung erzielt, während die dünneren Randbereiche ein glattes und unauffälliges Erscheinungsbild ermöglichen. Alternativ kann das Blickschutzelement jedoch auch flach ausgebildet sein und insbesondere eine im Wesentlichen einheitliche Materialstärke aufweisen.

Der Grundkörper weist bevorzugt eine Längserstreckung entlang einer Längsrichtung auf, welche größer ist als die übrigen Dimensionen des Grundkörpers. Das Blickschutzelement weist bevorzugt eine Längserstreckung entlang einer Längsrichtung auf, welche größer ist als die übrigen Dimensionen des Blickschutzelements. Das Blickschutzelement ist bevorzugt entlang der Längsrichtung des Grundkörpers angeordnet, insbesondere im Wesentlichen parallel dazu. Vorteilhafterweise ermöglicht die parallele Anordnung des Blickschutzelements entlang der Längsrichtung des Grundkörpers eine besonders stabile und gleichmäßige Integration, wodurch Materialversatz oder Faltenbildung vermieden werden. Zudem wird die Anpassung an die natürliche Körperform erleichtert, was sowohl die Funktionalität als auch den Tragekomfort verbessert.

Vorzugsweise umfasst das Blickschutzelement ein Verstärkungselement und bevorzugt eine Vielzahl von Verstärkungselementen, besonders bevorzugt von baugleichen Verstärkungselementen. Das Verstärkungselement weist bevorzugt eine dritte elastische Verformbarkeit auf. Vorzugsweise weist das Verstärkungselement ein Elastizitätsmodul von 10 MPa bis 30 MPa auf. Vorteilhafterweise gewährleistet das Verstärkungselement eine optimale Kombination aus Flexibilität und Formstabilität, wobei eine gezielte Verformung unter Druck und eine Rückkehr in die ursprüngliche Form erreicht wird, ohne den Tragekomfort negativ zu beeinträchtigen. Besonders vorzugsweise weist das Verstärkungselement ein Elastizitätsmodul von 10 MPa bis 20 MPa auf. Vorteilhafterweise ist das Verstärkungselement besonders für die alltägliche Anwendung geeignet und weist einen hohen Tragekomfort auf. Besonders vorzugsweise weist das Verstärkungselement ein Elastizitätsmodul von 20 MPa bis 30 MPa auf. Vorteilhafterweise ist das Verstärkungselement besonders für Sportbekleidung oder den Arbeitsschutz geeignet. Sämtliche zuvor genannten Wertebereiche gewährleisten in förderlicher Weise eine optimale Kombination aus Formstabilität und Flexibilität und sind insbesondere besonders an einen bestimmten Verwendungszweck angepasst. Durch die anwendungsabhängige Wahl des Elastizitätsmoduls kann vorteilhafterweise der Tragekomfort optimiert und gleichzeitig eine langlebige, formtreue Nutzung in unterschiedlichen Einsatzbereichen - von Alltagsbekleidung bis hin zu Sportbekleidung und Arbeitsschutzbekleidung - gewährleistet werden.

Vorzugsweise ist die dritte elastische Verformbarkeit größer als die erste elastische Verformbarkeit. Besonders vorzugsweise ist die dritte elastische Verformbarkeit größer als die zweite elastische Verformbarkeit. Alternativ sind die dritte elastische Verformbarkeit und die zweite elastische Verformbarkeit im Wesentlichen gleich. Das Verstärkungselement kann aus dem gleichen Material wie das Blickschutzelement bestehen. Alternativ besteht das Verstärkungselement aus einem anderen Material als das Blickschutzelement, insbesondere aus einem steiferen Material. Bevorzugt ist das Verstärkungselement bei bestimmungsgemäßer Anwendung nicht knickbar und/oder faltbar. Das Verstärkungselement weist bevorzugt eine Längserstreckung entlang einer Längsrichtung auf, welche größer ist als die übrigen Dimensionen des Verstärkungselements. Das Verstärkungselement ist bevorzugt quer zu dem Blickschutzelement angeordnet, insbesondere derart, dass die Längsrichtung des Verstärkungselements im Wesentlichen senkrecht zu der Längsrichtung des Blickschutzelements ist. Das Verstärkungselement kann alternativ auch längs oder diagonal zur Längsrichtung des Blickschutzelements angeordnet werden. Vorteilhafterweise lässt sich durch die relative Orientierung des Verstärkungselements eine auf den jeweiligen Anwendungsfall angepasste optimale Unterstützung einstellen. Das Blickschutzelement und/oder das Verstärkungselement ist bevorzugt nicht auf eine lineare oder rechteckige Geometrie beschränkt, sondern kann in vielfältigen, auch nichtlinearen oder periodischen Strukturen ausgeführt sein. Bevorzugt kann das Blickschutzelement und/oder das Verstärkungselement vorzugsweise eine dreieckige, trapezförmige, prismatische, rhombische, parallelogrammförmige, polygonale, wellenförmige, sinusförmige, meanderförmige, zick-zack-förmige, spiralförmige oder ringförmige Gestalt aufweisen. Ferner sind bevorzugt kreisförmige, ellipsenförmige, bogenförmige, halbkreisförmige oder segmentbogenförmige Ausführungen umfasst. Ebenso können vorzugsweise hexagonale, wabenförmige, netzartige, gitterartige oder rasterförmige Ausführungsformen vorgesehen sein. Weiterhin sind beispielsweise sternförmige, kreuzförmige, X- oder Y-förmige, T-förmige oder hakenförmige Geometrien möglich. Auch fraktale, hierarchisch aufgebaute oder unregelmäßig polygonale Formen sind vorzugsweise eingeschlossen, ebenso wie topologisch offene oder geschlossene Profile. Bei weiteren Ausführungsformen kann das Blickschutzelement und/oder das Verstärkungselement eine kombinierte Geometrie aus mehreren der vorgenannten Formen aufweisen, um die mechanischen Eigenschaften gezielt einzustellen. Vorteilhafterweise lässt sich durch die gezielte Kombination von Materialeigenschaften und Anordnung des Verstärkungselements eine dynamische Balance zwischen Stabilität und Flexibilität erzielen, sodass das Blickschutzelement einerseits zuverlässig ein glattes Aussehen bietet und sich andererseits angenehm an die Tragesituation anpasst. Gleichzeitig erlaubt die Ausrichtung des Verstärkungselements - quer, längs oder diagonal - eine feine Abstimmung der Unterstützung genau dort, wo sie benötigt wird, wodurch Komfort und Funktionalität optimiert sind.

Bevorzugt ist das Verstärkungselement in oder an dem Kernbereich des Blickschutzelements angeordnet. Die Verstärkungselemente können horizontal, und/oder vertikal, und/oder quer, und/oder überkreuz, und/oder übereinanderliegend angeordnet sein. Vorteilhafterweise erlaubt das Verstärkungselement die mechanischen Eigenschaften, im Speziellen die Verformbarkeit, des Blickschutzelements anzupassen und kann insbesondere für eine lokale Verstärkung, insbesondere einer Querversteifung, sorgen. Beispielsweise kann eine Rippenstruktur der Verstärkungselemente entlang der Längsrichtung die Stabilität bei intensiver Bewegung erhöhen, wie insbesondere beim Sport. Vorteilhafterweise kann ein Kreuzmuster der Verstärkungselemente für eine gleichmäßige Druckverteilung und einen besonders hohen Tragekomfort im Alltag sorgen.

Bevorzugt umfasst das Blickschutzelement einen Hauptbereich und einen Nebenbereich, welcher in Längsrichtung an dem Hauptbereich angeordnet und damit besonders bevorzugt verbunden ist. Der Hauptbereich ist in einer Quererstreckung senkrecht zur Längsrichtung des Blickschutzelements breiter als der Nebenbereich. Vorzugsweise kann eine Längserstreckung des Nebenbereichs entlang der Längsrichtung des Blickschutzelements länger sein als die Längserstreckung des Hauptbereichs. Der Hauptbereich ist bevorzugt ovalförmig. Der Nebenbereich ist vorzugsweise an einem dem Hauptbereich gegenüberliegenden Ende abgerundet. Das Verstärkungselement ist bevorzugt in dem Nebenbereich und/oder dem Kernbereich angeordnet. Vorteilhafterweise erlaubt die Unterteilung des Blickschutzelements in den Hauptbereich und den Nebenbereich eine gezielte Steuerung von Form und Verformbarkeit. Dabei kann der Hauptbereich bevorzugt Stabilität bieten und der Nebenbereich zusätzliche Flexibilität zur Anpassung an das Hygieneprodukt oder das Bekleidungsstück ermöglichen. Durch die bevorzugte Anordnung des Verstärkungselements in dem Nebenbereich oder dem Kernbereich kann die mechanische Unterstützung punktgenau angepasst werden, was sowohl den Schutz als auch den Tragekomfort optimiert.

Bei einer bevorzugten Weiterbildung, bei welcher der Grundkörper zumindest die erste Schicht und die zweite Schicht aufweist, ist das Blickschutzelement zwischen der ersten Schicht und der zweiten Schicht angeordnet, bevorzugt innerhalb des Grundkörpers. Vorteilhafterweise ist das Blickschutzelement besonders geschützt und stabilisiert angeordnet.

Bei einer bevorzugten Weiterbildung ist das Blickschutzelement an der kleidungsseitigen Oberfläche des Grundkörpers angeordnet. Dadurch wird vorteilhafterweise ein besonders hoher Tragekomfort bereitgestellt. Bevorzugt kommt das Blickschutzelement nicht in direkten Kontakt mit dem Genitalbereich der Person. Vorteilhafterweise gewährleistet die Anordnung des Blickschutzelements an der kleidungsseitigen Oberfläche dass die Hygiene erhöht wird. Vorzugsweise weist das Blickschutzelement zum Ankleben des Hygieneproduktes an dem Bekleidungsstück ein Haftmittel auf oder besteht aus einem haftenden Material. Alternativ kann das Blickschutzelement auch an der körperseitigen Oberfläche angeordnet sein. Vorteilhafterweise kann das Blickschutzelement durch Ankleben besonders schnell und günstig an dem Hygieneprodukt angeordnet und befestigt werden.

Vorzugsweise ist das Blickschutzelement an einem Ende des Grundkörpers angeordnet, insbesondere an dem Ende, welches bei bestimmungsgemäßer Verwendung des Hygieneprodukt einer Körpervorderseite der Person zugewandt ist. Bevorzugt bedeckt das Blickschutzelement mindestens 15% der Fläche des Grundkörpers. Vorzugsweise bedeckt das Blickschutzelement weniger als 85% der Fläche des Grundkörpers. Bevorzugt bedeckt das Blickschutzelement von 20% bis 70% der Fläche des Grundkörpers, besonders bevorzugt von 30% bis 50%, höchst bevorzugt von 30% bis 40%. Vorteilhafterweise bilden diese Wertebereiche eine optimierte Balance zwischen einem geringen Materialeinsatz und der effizienten Bereitstellung des glatten Aussehens.

Bei einer bevorzugten Weiterbildung weist das Blickschutzelement eine Vielzahl von Einkerbungen oder Zwischenräumen auf. Vorzugsweise sind die Einkerbungen in dem Randbereich tiefer als in dem Kernbereich. Bevorzugt sind die Zwischenräume in dem Randbereich größer als in dem Kernbereich. Die Einkerbungen oder Zwischenräume können bevorzugt kreisförmig oder quadratisch oder rillenförmig ausgebildet sein. Ebenso ist eine Kombination dieser Formen bevorzugt. Vorteilhafterweise ist das Blickschutzelement in dem Randbereich verformbarer als in dem Kernbereich, wodurch besonders vorteilhafterweise ein hoher Tragekomfort und ein vorteilhaftes glattes Aussehen resultieren. Alternativ kann das Blickschutzelement jedoch auch eine glatte und insbesondere unstrukturierte Oberfläche aufweisen. Vorteilhafterweise führen die Einkerbungen und Zwischenräume in dem Randbereich zu einer erhöhten Verformbarkeit, sodass sich das Blickschutzelement besonders komfortabel an die Konturen des Genitalbereichs anschmiegt. Gleichzeitig wird durch die Gestaltung von Kernbereich und Randbereich ein glattes, ästhetisches Erscheinungsbild gewährleistet. Bevorzugt bilden die Einkerbungen oder die Zwischenräume ein hexagonales Gitter aus. Dadurch lassen sich die mechanischen Eigenschaften des Blickschutzelements, wie beispielsweise die Verformbarkeit besonders effizient und vielseitig einstellen. Bevorzugt bilden die Einkerbungen oder die Zwischenräume ein wellenförmiges Muster, und/oder ein längsrillenförmiges Muster, und/oder ein querrillenförmiges Muster aus. Dadurch lassen sich die mechanischen Eigenschaften des Blickschutzelements, wie beispielsweise die Verformbarkeit und die Verteilung von Zug- oder Druckeinwirkungen besonders effizient verteilen und vielseitig einstellen.

Bei einer bevorzugten Weiterbildung umfasst das Blickschutzelement ein Silikon oder besteht aus Silikon. Vorteilhafterweise kann Silikon besonders effizient in fluider Form eingesetzt werden, wobei es durch Feuchtigkeit und/oder Wärme besonders einfach und kostengünstig in relativ kurzer Zeit verfestigt werden kann. Silikon ist zudem besonders hautfreundlich und wirkt vorteilhafterweise flüssigkeitsabweisend, wodurch es sich besonders für den Einsatz als zusätzliche Barriere gegen die Weiterleitung von Flüssigkeit zur Bekleidung eignet.

Bei einer bevorzugten Weiterbildung umfasst das Blickschutzelement ein Polyurethan oder besteht aus Polyurethan. Vorteilhafterweise ist Polyurethan besonders robust und langlebig. Zudem lässt sich Polyurethan in fluider Form einsetzen und durch die Anwesenheit von Feuchtigkeit oder bestimmter Chemikalien schnell verfestigen. Vorteilhafterweise sind Materialien wie Silikon oder Polyurethan hautfreundlich und für den direkten Kontakt mit der Haut geeignet. Sie sind dermatologisch erprobt und minimieren das Risiko von Irritationen oder allergischen Reaktionen der Haut. Zusätzlich können die Materialien durch nachhaltige Herstellungsverfahren, beispielsweise als recycelbares thermoplastisches Polyurethan oder als biologisch abbaubare Polymere, zur Umweltfreundlichkeit beitragen.

Bei einer bevorzugten Weiterbildung umfasst das Blickschutzelement ein Epoxidharz oder besteht aus Epoxidharz. Vorteilhafterweise lässt sich Epoxidharz insbesondere durch ein Zweikomponenten System einfach einsetzen und aushärten. Zudem weist Epoxidharz eine vorteilhaft hohe Haftung und Formstabilität auf.

Bei einer bevorzugten Weiterbildung umfasst das Blickschutzelement ein UV-härtendes Polymer oder besteht aus dem UV-härtendem Polymer. Vorteilhafterweise können UV-härtende Polymere durch UV-Licht innerhalb von wenigen Sekunden bis Minuten ausgehärtet werden und sind im Hinblick auf ihre mechanischen Eigenschaften, wie insbesondere der Verformbarkeit, besonders vielseitig einstellbar. Zudem sind viele UV-härtende Polymere bereits medizinisch erprobt. Bei einer bevorzugten Weiterbildung umfasst das Blickschutzelement ein Wachs, insbesondere ein Mischwachs und/oder Paraffin oder besteht daraus. Vorteilhafterweise kann erwärmtes Wachs durch Abkühlen besonders einfach, kostengünstig und schnell verfestigt werden. Ferner ist das Wachs besonders gut hautverträglich. Bei einer bevorzugten Weiterbildung umfasst das Blickschutzelement ein thermoplastisches Polymer, insbesondere ein thermoplastisches Olefin. Vorteilhafterweise kann das erwärmte thermoplastische Polymer im fluiden Zustand besonders effizient eingesetzt werden und durch simples Abkühlen besonders unkompliziert, kostengünstig und schnell verfestigt werden. Bei einer bevorzugten Weiterbildung umfasst das Blickschutzelement ein Hydrogel oder besteht aus dem Hydrogel. Vorteilhafterweise können Hydrogele in flüssiger Form einfach eingesetzt werden, sind besonders flexibel und quellen mit Flüssigkeiten, sodass Hydrogele eine bestimmte Menge an Flüssigkeit binden können.

Bei einer bevorzugten Weiterbildung umfasst das Blickschutzelement Latex oder besteht aus Latex. Vorteilhafterweise ist Latex besonders elastisch, medizinisch erprobt und biologisch abbaubar. Bei einer bevorzugten Weiterbildung umfasst das Blickschutzelement ein biobasiertes Polymer oder besteht aus dem biobasierten Polymer. Vorteilhafterweise sind biobasierte Polymere besonders nachhaltig produzierbar und optional auch biologisch abbaubar. Bei einer bevorzugten Weiterbildung umfasst das Blickschutzelement Polyethylenterephthalat oder besteht aus Polyethylenterephthalat. Vorteilhafterweise ist Polyethylenterephthalat besonders gut recyclebar und mechanisch stabil. Bei einer bevorzugten Weiterbildung umfasst das Blickschutzelement Milchproteinfasern oder besteht aus Milchproteinfasern. Milchproteinfasern sind bevorzugt synthetisch hergestellte Fasern aus dem Milcheiweiß Kasein. Vorteilhafterweise ist das Blickschutzelement besonders nachhaltig und ökologisch produzierbar. Bei einer bevorzugten Weiterbildung umfasst das Blickschutzelement Viskose und/oder Seide oder besteht aus Viskose und/oder Seide. Vorteilhafterweise ist das Blickschutzelement biologisch produzierbar und besonders angenehm. Bei einer bevorzugten Weiterbildung umfasst das Blickschutzelement Polymilchsäure (PLA) oder ein PLA-Derivat oder besteht daraus. Vorteilhafterweise sind PLA und PLA-Derivate besonders nachhaltig produzierbar, medizinisch erprobt und biologisch abbaubar. Auch ihre thermische Stabilität ist vorteilhaft. Bei einer bevorzugten Weiterbildung umfasst das Blickschutzelement Chitosan oder ein funktionalisiertes Chitosan-Derivat oder besteht daraus. Vorteilhafterweise sind Chitosan oder funktionalisierte Chitosan-Derivate nachhaltig produzierbar und biologisch abbaubar. Vorzugsweise weist das Chitosan oder das funktionalisierte Chitosan-Derivat antibakterielle Eigenschaften auf und verbessert damit vorteilhafterweise die Hygiene.

Bei einer bevorzugten Weiterbildung umfasst das Blickschutzelement ein thermochromes Polymer oder besteht aus dem thermochromen Polymer. Durch den Einsatz eines thermochromen Polymers können vorteilhafterweise Temperaturänderungen durch einen Farbwechsel angezeigt werden. Bei einer bevorzugten Weiterbildung umfasst das Blickschutzelement ein selbstheilendes Polymer oder besteht aus dem selbstheilenden Polymer. Bevorzugt ist das selbstheilende Polymer in From von Mikrokapseln vorgesehen. Vorteilhafterweise weist das Blickschutzelement selbstreparierende Eigenschaften auf. Bei einer bevorzugten Weiterbildung umfasst das Blickschutzelement ein Aerogel, insbesondere auf Siliziumbasis, oder besteht aus dem Aerogel. Vorteilhafterweise ist das Aerogel besonders leicht und weist zudem eine hohe Absorption von Flüssigkeiten auf. Bei einer bevorzugten Weiterbildung umfasst das Blickschutzelement einen Vliesstoff oder besteht aus dem Vliesstoff. Vorteilhafterweise ist das Blickschutzelement besonders leicht, saugfähig und atmungsaktiv. Bei einer bevorzugten Weiterbildung umfasst das Blickschutzelement einen Schaumstoff, insbesondere einen Memory-Schaumstoff oder besteht daraus. Vorteilhafterweise ist das Blickschutzelement besonders leicht, wärmeisolierend, saugfähig und atmungsaktiv. Bei einer bevorzugten Weiterbildung umfasst das Blickschutzelement ein Gelkissen oder eine Gelplatte oder besteht daraus. Vorteilhafterweise sind Gelkissen und Gelplatten besonders hautfreundlich und im Hinblick auf ihre mechanischen Eigenschaften, wie insbesondere der Verformbarkeit, besonders vielseitig einstellbar. Bei einer bevorzugten Weiterbildung umfasst das Blickschutzelement eine Naturfasermatte und/oder eine Pflanzenfasermatte oder besteht darauf. Die Pflanzenfasermatte ist vorzugsweise eine Bambusfasermatte und/oder eine Hanffasermatte und/oder eine Bananenfasermatte und/oder eine Jutefasermatte und/oder eine Sisalfasermatte. Vorteilhafterweise sind Bambusfasermatten besonders nachhaltig, biologisch abbaubar und saugfähig. Bei einer bevorzugten Weiterbildung umfasst das Blickschutzelement Polyethylen oder besteht aus Polyethylen. Vorteilhafterweise ist Polyethylen besonders einfach maschinell einlegbar und feuchtigkeitsdicht. Bei einer bevorzugten Weiterbildung umfasst das Blickschutzelement ein thermoplastisches Elastomer oder besteht aus dem thermoplastischen Elastomer. Vorteilhafterweise ist das thermoplastische Elastomer besonders flexibel und wiederverwendbar. Bei einer bevorzugten Weiterbildung umfasst das Blickschutzelement Papier oder Karton oder besteht daraus. Vorteilhafterweise ist das Blickschutzelement biologisch abbaubar.

Bei einer bevorzugten Weiterbildung weist das Blickschutzelement eine Nanopartikel basierte Beschichtung auf. Diese Nanopartikel basierte Beschichtung hat bevorzugt antibakterielle Eigenschaften. Vorteilhafterweise wird dadurch die Hygiene verbessert. Außerdem kann die Nanopartikel basierte Beschichtung die Absorption von Flüssigkeiten erhöhen. Bei einer bevorzugten Weiterbildung umfasst das Blickschutzelement eine Silberionenbeschichtung auf. Vorteilhafterweise ist das silberionenbeschichtete Blickschutzelement antibakteriell und damit besonders hygienisch und geruchsneutral. Bei einer bevorzugten Weiterbildung umfasst das Blickschutzelement eine mit Graphen beschichtete Folie. Vorzugsweise ist die mit Graphen beschichtete Folie an einer Oberfläche des Blickschutzelements angeordnet. Die mit Graphen beschichtete Folie hat bevorzugt antibakterielle Eigenschaften. Vorteilhafterweise wird dadurch die Hygiene verbessert. Weiter vorteilhafterweise wird durch die mit Graphen beschichtete Folie eine hohe Wärmeleitfähigkeit bereitgestellt. Bei einer bevorzugten Weiterbildung umfasst das Blickschutzelement Baumwolle und/oder Wolle und/oder ein Textilmaterial oder besteht daraus. Vorteilhafterweise ist das Blickschutzelement atmungsaktiv und flexibel. Bei einer bevorzugten Weiterbildung umfasst das Blickschutzelement Nylon und/oder Elastan oder besteht daraus. Bei einer bevorzugten Weiterbildung umfasst das Blickschutzelement Leder oder besteht aus Leder. Vorteilhafterweise ist das Blickschutzelement einfach zu reinigen und wiederverwendbar. Bei einer bevorzugten Weiterbildung umfasst das Blickschutzelement ein Biopolymer, insbesondere aus Maisstärke oder Zuckerrohr, oder besteht daraus. Bevorzugt umfasst das Blickschutzelement eine Maisstärkefolie. Vorteilhafterweise ist das Blickschutzelement nachhaltig produzierbar, hautverträglich und biologisch abbaubar.

Bei einer bevorzugten Weiterbildung umfasst das Blickschutzelement ein Metall, insbesondere in dem Kernbereich und/oder in dem Verstärkungselement. Das Metall ist bevorzugt ein Metall aus der Gruppe: Aluminium, und/oder Kupfer, und/oder Edelstahl, und/oder Titan, und/oder Silber, und/oder Gold. Das Metall ist insbesondere am Stück, in Drahtform und/oder in Pulverform. Bevorzugt besteht das Verstärkungselement aus dem Metall. Bei einer bevorzugten Weiterbildung umfasst das Blickschutzelement, bevorzugt in dem Kernbereich und/oder in dem Verstärkungselement: Glas, und/oder Keramik, und/oder Naturstein, insbesondere Granit oder Marmor, und/oder Holz, und/oder Kork, und/oder einen Epoxid-basierten Verbundwerkstoff, und/oder Polyvinylchlorid, und/oder Polycarbonat, insbesondere jeweils am Stück und/oder in Pulverform. Bevorzugt besteht das Verstärkungselement aus einem der in dem vorhergehenden Satz genannten Materialien. Bei einer bevorzugten Weiterbildung umfasst das Blickschutzelement Sand und/oder Faserstoffe, insbesondere in dem Kernbereich und/oder in dem Verstärkungselement. Die Faserstoffe sind insbesondere Kohlenstofffasern, und/oder Glaswolle, und/oder Steinwolle. Vorteilhafterweise lassen sich die mechanischen Eigenschaften des Blickschutzelements durch derartige Zusätze einfach und teilweise auch kosteneffizient einstellen. Bei einer bevorzugten Weiterbildung umfasst das Blickschutzelement einen Silberfaserstoff, und/oder einen silberbeschichteten Baumwollstoff, und/oder ein Silbernetz, und/oder ein Silberjersey. Silberhaltige Materialien dienen vorteilhafterweise zur verbesserten Hygiene und zur Geruchsneutralisierung. Bei einer bevorzugten Weiterbildung umfasst das Blickschutzelement eine Goldfolie und/oder Goldfäden, wobei die Goldfäden bevorzugt in ein Textilmaterial des Blickschutzelements eingearbeitet sind. Bei einer bevorzugten Weiterbildung umfasst das Blickschutzelement eine antibakterielle Beschichtung. Vorzugsweise umfasst das Blickschutzelement eine enzymatische Beschichtung und/oder eine mikrobielle Beschichtung. Dadurch kann das Blickschutzelement zu einem verbesserten Mikrobiom im Genitalbereich beitragen und insbesondere unangenehme Gerüche verhindern. Bei einer bevorzugten Weiterbildung umfasst das Blickschutzelement Kohle oder Aktivkohle. Vorteilhafterweise ist das Blickschutzelement geruchsneutralisierend.

Bei einer bevorzugten Weiterbildung ist das Blickschutzelement in zumindest einem Querschnitt, bevorzugt in allen Querschnitten kreisrund und/oder oval. Vorteilhafterweise ist das Blickschutzelement besonders anschmiegsam und bequem zu tragen. Bei einer bevorzugten Weiterbildung ist das Blickschutzelement in zumindest einem Querschnitt, bevorzugt in allen Querschnitten viereckig, insbesondere rechteckig oder quadratisch, oder dreieckig, oder polygon. Vorteilhafterweise ist das Blickschutzelement besonders einfach und günstig produzierbar. Bei einer bevorzugten Ausführungsform werden die Verfahrensschritte in der Reihenfolge ihrer Nennung in dem unabhängigen Verfahrensanspruch und/oder in der Reihenfolge ihrer Nennung in dieser Beschreibung aufeinanderfolgend durchgeführt. Bei Versuchen wurde festgestellt, dass sich durch sämtliche zuvor genannte Merkmale und/oder Merkmalskombinationen mehrere vorteilhafte Effekte ergeben, welche der Verbesserung der Gegenstände der unabhängigen Ansprüche zuträglich sind. Die Vorteile ergeben sich insbesondere aus der Beschreibung, auch wenn sie aus Gründen der Übersichtlichkeit nicht bei allen Merkmalen oder Merkmalskombinationen wiederholt wurden. Weitere Vorteile der Erfindung werden von einer Fachperson erkannt. Es versteht sich, dass die Gegenstände der unabhängigen Ansprüche oder der vorstehenden Beschreibung ähnliche und/oder identische Weiterbildungen aufweisen. Bei einer bevorzugten Ausführungsform ist eine beliebige Kombination der abhängigen Ansprüche und/oder der in der Beschreibung beschriebenen Merkmale mit dem jeweiligen unabhängigen Anspruch vorgesehen.

Weitere Eigenschaften und Vorteile der Erfindung ergeben sich aus der nachfolgenden Beschreibung anhand von Ausführungsbeispielen und anhand der Zeichnungen. Obwohl die Erfindung in den Figuren und der vorstehenden Beschreibung detailliert dargestellt und offenbart ist, sind diese Darstellungen und Beschreibungen als rein illustrativ oder beispielhaft und nicht als einschränkend anzusehen. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind.
- Figur 1: zeigt schematisch ein herkömmliches Hygieneprodukt;
- Figur 2: zeigt schematisch ein Blickschutzelement mit einem Verstärkungselement in einem Randbereich;
- Figur 3: zeigt schematisch ein Blickschutzelement mit Verstärkungselementen in einem Hauptbereich;
- Figur 4: zeigt schematisch ein Blickschutzelement mit zick-zack förmigen Verstärkungselementen;
- Figur 5: zeigt schematisch ein Blickschutzelement mit einem wabenförmigen förmigen Verstärkungselement;
- Figur 6: zeigt schematisch ein Hygieneprodukt mit Blickschutzelement;
- Figur 7: zeigt schematisch ein Hygieneprodukt mit Blickschutzelement in einem Längsschnitt;
- Figur 8: zeigt schematisch ein Hygieneprodukt mit einem Blickschutzelement, welches eine Vielzahl von Verstärkungselementen aufweist, in einem Längsschnitt;
- Figur 9: zeigt schematisch das Hygieneprodukt der Figur 8 in einem Querschnitt;
- Figur 10: zeigt schematisch die Silhouette einer Person, welche ein herkömmliches Hygieneprodukt unter der Bekleidung trägt;
- Figur 11: zeigt schematisch die Silhouette einer Person, welche ein Blickschutzelement oder ein Hygieneprodukt mit Blickschutzelement unter der Bekleidung trägt.

In den Figuren verwendete gleiche Bezugszeichen bezeichnen gleiche oder zumindest gleichwirkende Elemente. Die Begriffe "oben", "unten", "links" und "rechts" sowie daraus abgeleitete richtungsabhängige Angaben, wie beispielsweise "Oberseite", beziehen sich auf die Schreibrichtung/Leserichtung der zu einer Zeichnung zugehörigen Figurenbezeichnung "Fig.", welche unter der Zeichnung auf die Zeichenebene gedruckt ist. Dabei ist die horizontale Richtung parallel zur Schreibrichtung von "Fig." und die vertikale Richtung steht senkrecht zur Schreibrichtung von "Fig". Der Schreibrichtung wird eine waagerechte rechtsläufige Schrift, das heißt, primär von links nach rechts wie beispielsweise im Lateinischen, Englischen, Französischen und Deutschen zugrunde gelegt.

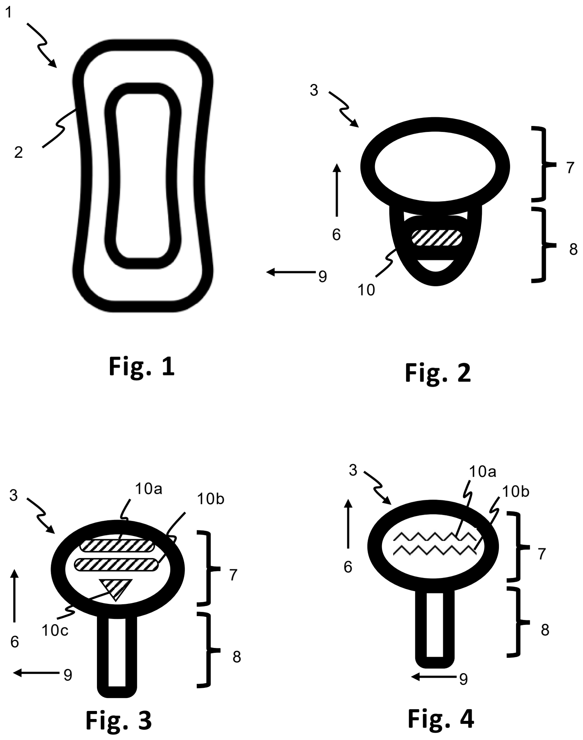

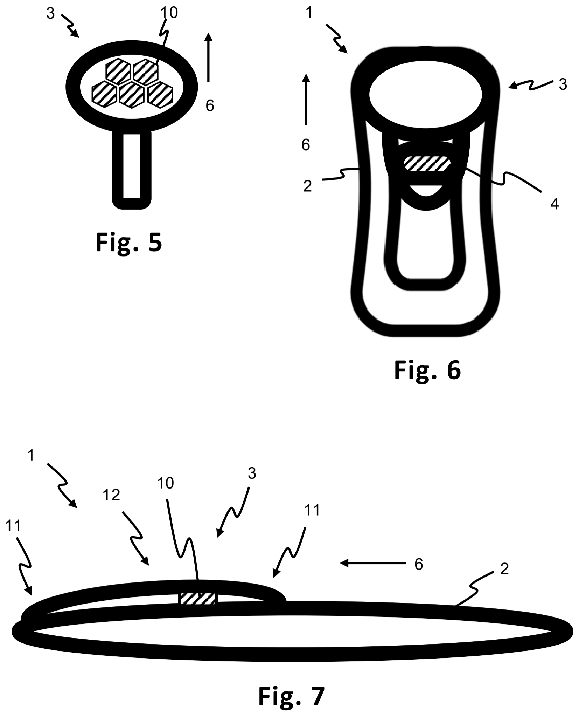

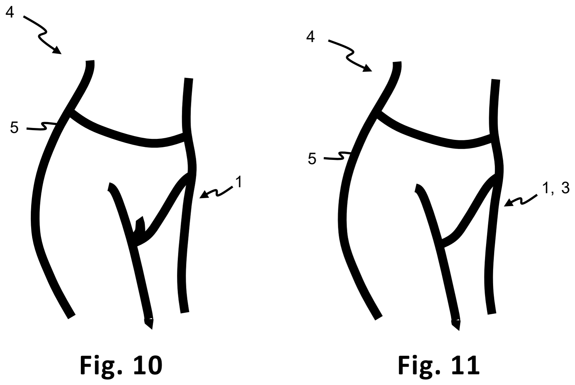

Figur 1 zeigt schematisch ein herkömmliches Hygieneprodukt 1, im Speziellen eine Slipeinlage. Das Hygieneprodukt 1 weist einen elastisch verformbaren Grundkörper 2 auf, aber kein Blickschutzelement 3 (siehe Fig. 2 bis 4). Das herkömmliche Hygieneprodukt 1 passt sich eng an die Körperform einer Person 4 (siehe Fig. 5a) an, sodass sich unvorteilhaft Konturen des Genitalbereichs der Person 4 bei enganliegenden Bekleidungsstücken 5 (siehe Fig 5a) abzeichnen. Figur 2 zeigt schematisch ein Blickschutzelement 3, welches dazu ausgebildet ist, an dem Hygieneprodukt 1 (siehe Fig. 6) angebracht zu werden. Das Blickschutzelement 3 weist eine zweite elastische Verformbarkeit auf, die geringer ist als eine erste elastische Verformbarkeit des Hygieneprodukts 1 (siehe Fig. 1 und 3). Das Blickschutzelement 3 ist also insbesondere steifer als das Hygieneprodukt 1. Das Blickschutzelement 3 ist dazu ausgebildet, bei dem Tragen an dem Bekleidungsstück 5 (siehe Fig. 11) oder an dem Hygieneprodukt 1, ein bei Draufsicht auf das Bekleidungsstück 5 glattes Aussehen über dem Genitalbereich einer Person 4 bereitzustellen (siehe Fig. 11). Das Blickschutzelement 3 weist eine Längserstreckung entlang einer Längsrichtung 6 auf. Das Blickschutzelement 3 umfasst zudem einen Hauptbereich 7 und einen Nebenbereich 8, welcher in Längsrichtung 6 unterhalb des Hauptbereichs 7 angeordnet ist. Der Hauptbereich 7 ist in einer Quererstreckung entlang einer Querrichtung 9 breiter als der Nebenbereich 8. Vorzugsweise ist der Nebenbereich 8 in einer Längserstreckung entlang der Längsrichtung 6 etwas länger als der Hauptbereich 7. Der Hauptbereich 7 ist ovalförmig. Der Nebenbereich 8 ist an seinem unteren Ende abgerundet. Das Blickschutzelement 3 umfasst ein Verstärkungselement 10. Das Verstärkungselement 10 weist eine dritte elastische Verformbarkeit auf, welche größer ist als die zweite elastische Verformbarkeit des Blickschutzelements 3, insbesondere in dem umgebenden Nebenbereich 8. Das Verstärkungselement 10 ist in Querrichtung 9 senkrecht zur Längserstreckung des Blickschutzelements 3 angeordnet und sorgt vorteilhafterweise für eine Querverstärkung.

Figur 3 zeigt schematisch ein Blickschutzelement 3 mit einer Vielzahl von Verstärkungselementen 10, welche in einem Hauptbereich 7 des Blickschutzelements 3 angeordnet sind. Die Vielzahl von Verstärkungselementen 10 umfasst ein erstes Verstärkungselement 10a, welches sich in Querrichtung 9 erstreckt, ein zweites Verstärkungselement 10b, welches sich ebenfalls in Querrichtung 9 erstreckt und ein drittes Verstärkungselement 10c, welches dreieckförmig ausgebildet ist. Das erste Verstärkungselement 10a und das zweite Verstärkungselement 10b können prismatisch ausgebildet sein. Figur 4 zeigt schematisch ein Blickschutzelement 3 mit zick-zack förmigen Verstärkungselementen 10a, 10b, welche sich in Querrichtung 9 erstrecken. Figur 5 zeigt schematisch ein Blickschutzelement 3 mit einem wabenförmigen Verstärkungselement 10. Figur 6 zeigt ein Hygieneprodukt 1 mit einem Blickschutzelement 3, insbesondere das Hygieneprodukt 1 der Fig. 1 mit dem Blickschutzelement 3 der Fig. 2. Das Blickschutzelement 3 ist in Längsrichtung 6 in dem oberen Bereich des Grundkörpers 2 angeordnet. Bevorzugt bedeckt das Blickschutzelement 3 etwa das obere Drittel des Grundkörpers 2. Das Hygieneprodukt 1 ist durch das Blickschutzelement 3 dazu ausgebildet ein bei Draufsicht auf das Bekleidungsstück 5 (siehe Fig. 5b) glattes Aussehen über dem Genitalbereich einer Person 4 bereitzustellen (siehe Fig. 5b). Das Blickschutzelement 3 ist an einer kleidungsseitigen Oberfläche des Grundkörpers 2 angeordnet. Das Blickschutzelement 3 kommt dadurch vorteilhafterweise nicht in direkten Kontakt mit dem Genitalbereich der Person 4. Das Hygieneprodukt 1 wird an dem Bekleidungsstück 5 einfach angeklebt, wobei das Blickschutzelement 3 dafür ein Haftmittel aufweist.

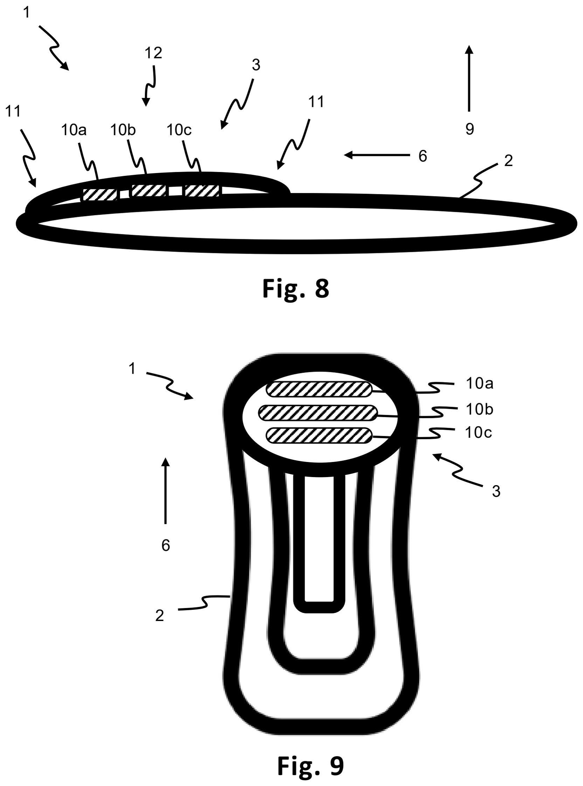

Figur 7 zeigt ein Hygieneprodukt 1 mit einem Blickschutzelement 3, insbesondere das Hygieneprodukt 1 mit dem Blickschutzelement 3 der Fig. 3 in einem Längsschnitt entlang der Längsrichtung 6 durch die Mitte des Hygieneprodukts 1. Das Blickschutzelement 3 weist einen Randbereich 11 und einen Kernbereich 12 auf, wobei der Randbereich 11 den Kernbereich 12 umschließt. Der Kernbereich 12 weist eine Materialstärke auf, welche größer ist als eine Materialstärke des Randbereichs 11. Dadurch ist das Blickschutzelement in dem Randbereich 11 verformbarer als in dem Kernbereich 12. Das Verstärkungselement 10 ist innerhalb des Kernbereichs 12 angeordnet. Dadurch lässt sich ein besonders hoher Tragekomfort und ein vorteilhaftes glattes Aussehen erreichen. Figur 8 zeigt schematisch ein Hygieneprodukt 1 mit einem Blickschutzelement 3 in einem Längsschnitt. Das Blickschutzelement 3 weist eine Vielzahl von Verstärkungselementen 10 auf. Die Vielzahl von Verstärkungselementen 10 umfasst ein erstes Verstärkungselement 10a, ein zweites Verstärkungselement 10b und ein drittes Verstärkungselement 10c, wobei das zweite Verstärkungselement 10b in Längsrichtung 6 zwischen dem ersten Verstärkungselement 10a und dem dritten Verstärkungselement 10c angeordnet ist. Bei einem anderen, hier nicht gezeigten, Ausführungsbeispiel ist das zweite Verstärkungselement 10b in Querrichtung 9 zwischen dem ersten Verstärkungselement 10a und dem dritten Verstärkungselement 10c angeordnet. Die Verstärkungselemente 10a, 10b, 10c können horizontal oder vertikal, quer, überkreuz, und/oder übereinanderliegend angeordnet sein. Die Verstärkungselemente 10 können aus Metalldrähten gebildet werden, welche in Silikon eingebettet sind. Alternativ oder ergänzend können die Verstärkungselemente 10 aus einem nicht knickbaren Polymer gebildet sein. Figur 9 zeigt schematisch das Hygieneprodukt 1 aus Fig. 8 mit einem Blickschutzelement 3, welches eine Vielzahl von Verstärkungselementen 10 aufweist, in einem Querschnitt.

Figur 10 zeigt die Silhouette einer Person 4, welche ein herkömmliches Hygieneprodukt 1, insbesondere jenes aus Fig. 1, unter der Bekleidung 5 trägt. Das herkömmliche Hygieneprodukt 1 passt sich eng an die Körperform der Person 4 an, sodass sich unvorteilhaft Konturen des Genitalbereichs der Person 4 bei dem enganliegenden Bekleidungsstück 5 abzeichnen. Figur 11 zeigt die Silhouette einer Person 4, welche ein Blickschutzelement 3, insbesondere das Blickschutzelement 3 aus Fig. 2, oder ein Hygieneprodukt 1 mit Blickschutzelement 3, insbesondere jenes aus Fig. 3, unter der Bekleidung 5 trägt. Durch den direkten Vergleich zwischen Fig. 6 und Fig. 7 lässt sich einfach erkennen, dass die Verwendung des Blickschutzelements 3 für ein glattes Aussehen im Genitalbereich der Person 4 in Fig. 7 sorgt.

Die in der vorstehenden Beschreibung, den Ansprüchen, den Ausführungsbeispielen und den Figuren beschriebenen oder gezeigten Merkmale können einzeln oder in beliebiger Kombination zur Verwirklichung der verschiedenen Ausführungsformen der Erfindung von Bedeutung sein. Bei der Lektüre der vorliegenden Offenbarung werden fachübliche weitere Modifikationen der Erfindung nahegelegt. Solche Modifikationen können andere Merkmale umfassen, die aus dem Stand der Technik bereits bekannt sind und die anstelle von oder zusätzlich zu den hier bereits beschriebenen Merkmalen verwendet werden können. Modifikationen der offenbarten Erfindung und deren Ausführungsformen können anhand der Zeichnungen, der Offenbarung und der Ansprüche verstanden und implementiert werden.

### Bezugszeichen

- 1: Hygieneprodukt
- 2: Grundkörper
- 3: Blickschutzelement
- 4: Person
- 5: Bekleidungsstück
- 6: Längsrichtung
- 7: Hauptbereich
- 8: Nebenbereich
- 9: Querrichtung
- 10: Verstärkungselement
- 11: Randbereich
- 12: Kernbereich

## Patentansprüche

1. Hygieneprodukt (1), insbesondere eine Monatsbinde oder eine Slipeinlage, zum Tragen an einem Bekleidungsstück (5), welches den Genitalbereich einer Person (4) bedeckt, insbesondere an Unterwäsche, umfassend
einen Grundkörper (2), welcher eine erste elastische Verformbarkeit aufweist, und ein Blickschutzelement (3), welches eine zweite elastische Verformbarkeit aufweist, die geringer ist als die erste elastische Verformbarkeit,
wobei das Hygieneprodukt (1) dazu ausgebildet ist, bei dem Tragen an dem Bekleidungsstück (5), ein bei Draufsicht auf das Bekleidungsstück (5) glattes Aussehen über dem Genitalbereich bereitzustellen.

2. Hygieneprodukt (1) nach Anspruch 1, bei dem das Blickschutzelement (3) einen Kernbereich (12) mit einer ersten Materialstärke und einen Randbereich (11) mit einer zweiten Materialstärke aufweist, wobei der Randbereich (11) den Kernbereich (12) zumindest teilweise oder vollständig umschließt und die erste Materialstärke größer ist als die zweite Materialstärke.

3. Hygieneprodukt (1) nach Anspruch 1 oder 2, bei dem der Grundkörper (2) zumindest eine erste Schicht und eine zweite Schicht aufweist und das Blickschutzelement (3) zwischen der ersten Schicht und der zweiten Schicht innerhalb des Grundkörpers (2) angeordnet ist.

4. Hygieneprodukt (1) nach Anspruch 1 oder 2, bei dem das Blickschutzelement (3) zum Ankleben des Hygieneproduktes (1) an dem Bekleidungsstück (5) an einer kleidungsseitigen Oberfläche des Grundkörpers (2) angeordnet ist und ein Haftmittel aufweist oder aus einem haftenden Material besteht.

5. Hygieneprodukt (1) nach einem der vorhergehenden Ansprüche, bei dem das Blickschutzelement (3) eine Vielzahl von Einkerbungen oder Zwischenräumen aufweist.

6. Hygieneprodukt (1) nach dem vorhergehenden Anspruch, bei dem die Einkerbungen oder die Zwischenräume ein hexagonales Gitter ausbilden.

7. Hygieneprodukt (1) nach einem der vorhergehenden Ansprüche, bei dem das Blickschutzelement (3) ein Silikon umfasst oder aus Silikon besteht.

8. Blickschutzelement (3) für ein Hygieneprodukt (1) nach einem der vorhergehenden Ansprüche und/oder für ein Bekleidungsstück (5), wobei die zweite elastische Verformbarkeit des Blickschutzelements (3) geringer ist als die erste elastische Verformbarkeit des Hygieneprodukts (1) und/oder geringer ist als eine elastische Verformbarkeit des Bekleidungsstücks (5).

9. Blickschutzelement (3) nach dem vorhergehenden Anspruch, welches ein wasserlösliches Material umfasst oder aus einem wasserlöslichen Material besteht.

10. Set umfassend ein Hygieneprodukt (1), insbesondere eine Monatsbinde oder eine Slipeinlage, und ein Blickschutzelement (3) gemäß einem der beiden vorhergehenden Ansprüche,
wobei das Set ein Befestigungselement umfasst, insbesondere einen Klebestreifen zur Befestigung des Blickschutzelements (3) an dem Hygieneprodukt (1) und/oder an einem Bekleidungsstück (5), und/oder
wobei das Blickschutzelement (3) an einer dem Hygieneprodukt (1) und/oder dem Bekleidungsstück (5) zuzuwendenden Oberfläche ein Haftmittel aufweist und/oder wobei das Blickschutzelement (3) aus einem haftenden Material besteht.

11. Verfahren zum Herstellen eines Hygieneprodukts (1) mit einem Blickschutzelement (3), insbesondere einer Monatsbinde oder einer Slipeinlage, mit den Schritten:
- Bereitstellen eines Grundkörpers (2)
- Einbringen oder Aufbringen eines fluiden Materials zwischen zwei Schichten des Grundkörpers (2) oder applizieren des fluiden Materials auf einer kleidungsseitigen Oberfläche des Grundkörpers (2)
- Ausbilden eines Blickschutzelements (3) durch Verfestigen des fluiden Materials.

12. Verwendung eines Hygieneprodukts (1), insbesondere einer Monatsbinde oder einer Slipeinlage, an einem Bekleidungsstück (5), welches den Genitalbereich einer Person (4) bedeckt, zum Bereitstellen eines bei Draufsicht auf das Bekleidungsstück (5) glatten Aussehens des Genitalbereichs.

13. Verwendung eines Blickschutzelements (3) an einem Hygieneprodukt (1), insbesondere einer Monatsbinde oder einer Slipeinlage, und/oder an einem Bekleidungsstück (5), welches den Genitalbereich einer Person (4) bedeckt, zum Bereitstellen eines bei Draufsicht auf das Bekleidungsstück (5) glatten Aussehens des Genitalbereichs.
